# EUROPEAN PATENT APPLICATION

(11) **EP 4 736 769 A1**
(43) Date of publication of application: **06.05.2026**
(21) Application number: 24832176.2
(22) Date of filing: 11.03.2024
(51) Int. Cl.: A61B 6/00, A61B 6/03, G06T 7/62, G06T 7/11

(54) **METHOD FOR CALCULATING DEMENTIA-RELATED INFORMATION USING VOLUME PREDICTED BY BRAIN CT AND ANALYSIS DEVICE THEREOF**

(30) Priority: 27.06.2023 KR 20230082852
(71) Applicant: Samsung Life Public Welfare Foundation, Seoul 04348 (KR)
(72) Inventor: PARK, Chae Jung, Seoul 06351 (KR); KIM, Soo Jong, Seoul 06351 (KR); GU, Yun A, Seoul 06351 (KR); SEO, Sang Won, Seoul 06351 (KR); JANG, Hye Min, Seoul 06351 (KR)
(74) Representative: Witte, Weller & Partner Patentanwälte mbB
(86) International application number: PCT/KR2024/003074
(87) International publication number: WO 2025/005388

(57) **Abstract**

A method for deriving dementia-related information using volume predicted from brain CT includes: a step of an analysis apparatus receiving a brain CT (Computed Tomography) image of a subject; a step of the analysis apparatus inputting the brain CT image into a pre-trained segmentation model to extract regions of interest; a step of the analysis apparatus inputting pixel information of the regions of interest into a pre-trained first learning model to predict the volume of at least one region among the regions of interest; and a step of the analysis apparatus inputting the volume of the at least one region into a pre-trained second learning model to derive dementia-related information of the subject.

## Description

### [Technical Field]

The following description relates to a technique for predicting dementia-related information of a subject using brain CT images.

### [Background Art]

Dementia refers to a syndrome that causes cognitive function impairment such as memory, language, and judgment. Alzheimer's disease is the most common type of dementia. Brain imaging such as MRI (Magnetic Resonance Imaging) and PET is used for Alzheimer's diagnosis.

### [Detailed Description of Invention]

### [Technical Problem]

3D (dimensional) MRI and PET-CT (positron emission tomography-Computed Tomography) require high time and cost for imaging. In contrast, CT has no restrictions such as the presence of metal substances in the human body, can be acquired much faster than MRI, and is relatively inexpensive.

The technology described below aims to provide a technique for calculating dementia-related information such as dementia status, dementia risk, and brain atrophy using only brain CT images.

### [Technical Solution]

In one general aspect, there is provided a method of calculating dementia-related information using volume predicted from brain CT including: a step of an analysis apparatus receiving a brain CT image of a subject; a step of the analysis apparatus inputting the brain CT image into a pre-trained segmentation model to extract regions of interest; a step of the analysis apparatus inputting pixel information of the regions of interest into a pre-trained first learning model to predict the volume of at least one region among the regions of interest; and a step of the analysis apparatus inputting the volume of the at least one region into a pre-trained second learning model to derive dementia-related information of the subject.

In another general aspect, there is provided analysis apparatus for calculating dementia-related information using volume predicted from brain CT including: an interface device for receiving a brain CT image of a subject; a storage device storing a segmentation model for extracting regions of interest from brain CT images, a first learning model for receiving brain region of interest information and predicting volume, and a second learning model for calculating dementia-related information; and a computing device that inputs the received brain CT image into the segmentation model to extract regions of interest, inputs pixel information of the extracted regions of interest into the first learning model to predict the volume of at least one region among the regions of interest, and inputs the volume of the at least one region into the second learning model to derive dementia-related information of the subject.

### [Effects of Invention]

The technology described below extracts regions of interest from CT images and derives dementia-related information based on the volume of the regions of interest. The technology described below can quickly and inexpensively derive significant dementia-related information using only CT images.

### [Brief Description of Drawings]

FIG. 1 is an example of a system for calculating dementia-related information based on CT images.
FIG. 2 is an example of a process for calculating dementia-related information using brain CT images.
FIG. 3 is an example of a process for training a segmentation model that extracts regions of interest from brain CT images.
FIG. 4 is an example of a training process for a learning model that predicts the volume of regions of interest based on extracted regions of interest.
FIG. 5 is an example of a training process for a learning model that derives dementia-related information based on brain region of interest volume.
FIG. 6 is an example of an analysis apparatus that derives dementia-related information of a subject using brain CT images.

### [Modes of the Invention]

The technology described below can be modified in various ways and can have various embodiments, and specific embodiments will be illustrated in the drawings and described in detail. However, this is not intended to limit the technology described below to specific embodiments, and should be understood to include all modifications, equivalents, and substitutes included in the spirit and technical scope of the technology described below.

Terms such as first, second, A, B, etc. may be used to describe various components, but the components are not limited by the terms and are used only for the purpose of distinguishing one component from another. For example, without departing from the scope of the technology described below, a first component may be referred to as a second component, and similarly, the second component may also be referred to as a first component. The term and/or includes a combination of a plurality of related described items or any of a plurality of related described items.

In the terms used in this specification, singular expressions should be understood to include plural expressions unless clearly interpreted differently in context, and terms such as "comprises" mean that the described features, numbers, steps, operations, components, parts, or combinations thereof exist, and should be understood not to exclude the possibility of the existence or addition of one or more other features, numbers, steps, operations, components, parts, or combinations thereof.

Before providing a detailed description of the drawings, it should be made clear that the division of components in this specification is merely divided by the main function that each component is responsible for. That is, two or more components to be described below may be combined into one component, or one component may be divided into two or more components according to more subdivided functions. In addition, each component to be described below may additionally perform some or all of the functions of other components in addition to its main function, and some of the main functions of each component may of course be exclusively performed by other components.

In addition, in performing a method or operation method, each process constituting the method may occur differently from the specified order unless a specific order is clearly stated in context. That is, each process may occur in the same order as specified, may be performed substantially simultaneously, or may be performed in reverse order.

The technology described below is a technology for calculating dementia-related information from brain CT.

Hereinafter, dementia includes Alzheimer's dementia.

Dementia-related information refers to information related to dementia diagnosis or dementia prediction. For example, dementia-related information may be at least one of information such as dementia onset status, dementia risk, dementia probability, dementia-related score, dementia prognosis prediction, degree of brain atrophy, beta-amyloid (amyloid-β, Aβ) positivity, tau protein positivity, and brain age.

Hereinafter, a device that analyzes brain CT to derive dementia-related information for a subject is referred to as an analysis apparatus. The analysis apparatus may take the form of a computer device such as a PC, a smart device, a network server, or a data processing dedicated chipset.

The analysis apparatus may derive dementia-related information based on brain images using multiple learning models. The analysis apparatus may extract region(s) of interest from CT using a segmentation model. In addition, the analysis apparatus may derive dementia-related information from CT or region(s) of interest using a classification model.

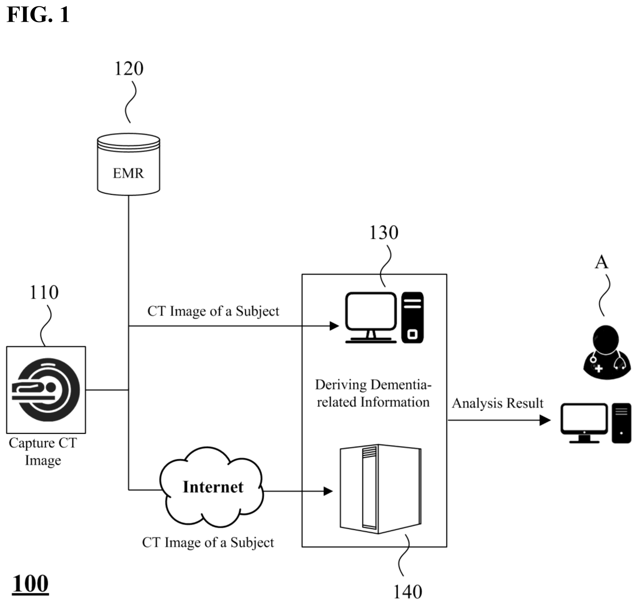

FIG.1 is an example of a system (100) for calculating dementia-related information based on CT images. In FIG. 1, the analysis apparatus is shown as a computer terminal (130) and a server (140).

The CT imaging device (110) captures a CT image of a subject. The CT image is an image of the brain region (brain CT image). The brain CT image generated by the CT imaging device (110) may be stored in a separate database such as EMR (Electronic Medical Record, 120).

The computer terminal (130) can receive the subject's brain CT image from the CT imaging device (110) or EMR (120) through a wired or wireless network. In some cases, the computer terminal (130) may be a device physically connected to the CT imaging device (110). The computer terminal (130) may uniformly preprocess the brain CT image.

The computer terminal (130) extracts region(s) of interest from the brain CT image. The computer terminal (130) may extract region(s) of interest from the brain CT image using a pre-trained segmentation model. The computer terminal (130) may estimate the volume of the region(s) of interest using a learning model. The computer terminal (130) may derive dementia-related information for the subject based on the volume of the region(s) of interest using a learning model. User A may check dementia-related information through the computer terminal (130).

The server (140) may receive the subject's brain CT image from the CT imaging device (110) or EMR (120) through a wireless network. The server (140) may uniformly preprocess the brain CT image.

The server (140) extracts region(s) of interest from the brain CT image. The server (140) may extract region(s) of interest from the brain CT image using a pre-trained segmentation model. The server (140) may estimate the volume of the region(s) of interest using a learning model. The server (140) may derive dementia-related information for the subject based on the volume of the region(s) of interest using a learning model. The server (140) may transmit the dementia-related information to a user terminal. User A may check dementia-related information through the user terminal.

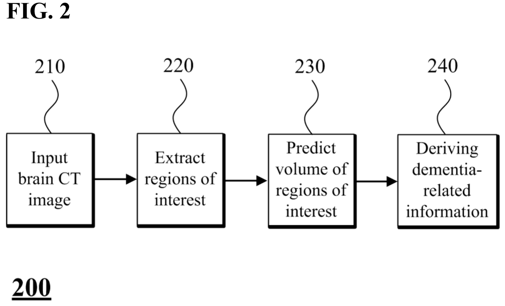

FIG. 2 is an example of a process (200) for calculating dementia-related information using brain CT images. The analysis apparatus may derive dementia-related information for a subject based on brain CT images.

The analysis apparatus receives a brain CT image of the subject (210). Brain CT may consist of multiple slices. The object analyzed by the analysis apparatus may be all of the multiple slices or at least one slice among the multiple slices.

The analysis apparatus may uniformly preprocess the brain CT image.

For example, the analysis apparatus may extract the entire brain region from the brain CT image. In this process, the analysis apparatus may use a pre-trained segmentation model. The analysis apparatus may extract the entire brain region from brain MRI images using the CIVET pipeline to generate ground truth, and train the segmentation model to extract the entire brain region from brain CT images based on the correct answer (entire brain region of brain MRI images). Thereafter, the analysis apparatus may extract the entire brain region from brain CT images using the pre-trained segmentation model.

In addition, the analysis apparatus may normalize the size or resolution of the subject's brain CT image to a certain size.

The analysis apparatus extracts regions of interest from the received brain CT image (220). The analysis apparatus may extract regions of interest using a pre- trained segmentation model. The analysis apparatus may extract multiple regions of interest using a segmentation model. The regions of interest correspond to regions for calculating the volume of the corresponding region.

The regions of interest may include at least one of a cerebrospinal fluid (CSF) region and a ventricle region. The regions of interest may include at least one of frontal CSF, temporal CSF, parietal CSF, occipital CSF, anterior lateral ventricle, posterior lateral ventricle, and peri-hippocampal CSF.

The analysis apparatus may estimate the volume of the extracted region(s) of interest (230). The analysis apparatus may estimate the volume of a specific region of interest using a learning model (e.g., regression analysis model) that has pre-trained the relationship between the number of pixels for a specific region of interest and the volume of the corresponding region. For this purpose, the analysis apparatus needs to normalize the size of the brain CT image or region of interest uniformly. The analysis apparatus may estimate the volume of a specific region of interest based on a uniformly normalized brain CT image. At this time, the learning model may be prepared in advance for each of multiple regions of interest. For example, the analysis apparatus may individually estimate the volume of the corresponding region using learning models for each of the frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region.

Meanwhile, the analysis apparatus may additionally use the subject's clinical information (gender, age, etc.) in addition to the brain CT image to estimate the volume of the region of interest.

The analysis apparatus may derive dementia-related information for the subject based on the volume of the region(s) of interest (240). The analysis apparatus may derive dementia-related information based on the volume of the region(s) of interest using a learning model. In addition, the analysis apparatus may derive the subject's dementia-related information by further using additional clinical information (age, gender, APOE4 genotype, etc.) in addition to the volume of the region(s) of interest.

Researchers constructed the aforementioned learning models using data from a population who visited an affiliated medical institution (Samsung Seoul Hospital). The population consisted of dementia patients (Alzheimer's disease dementia, ADD) and normal controls (NC). The population was selected as subjects who had both MRI and CT imaging within a certain period (1 year). The population excluded normal people who were too young and also excluded dementia patients with cardiovascular diseases. The population data includes MRI, CT, clinical information (gender, age, APOE4 phenotype), and dementia status (label value). The total number of subjects in the population was 895. Table 1 below shows information about the population. Researchers used data from 716 people (80% of 895 people) as training data and data from 179 people (20%) as test data. Validation was conducted with 5-fold cross validation.

**[Table 1]**

| | Total | NC | ADD |
|---|---|---|---|
| Subjects, N (%) | 895 (100.0) | 457 (51.1) | 438 (48.9) |
| Age, mean years (SD, Standard Deviation) | 67.4 (11.4) | 66.8 (12.5) | 68.0 (10.2) |
| Female, N (%) | 541 (60.4) | 279 (60.9) | 262 (59.8) |
| APOEε4 carriers, N(%) | 322 (36.0) | 86 (18.8) | 236 (51.5) |
| Frontal cerebrospinal fluid region, mean volume (mm³) | 61052.5 | 50380.9 | 72187.0 |
| Temporal cerebrospinal fluid region, mean volume (mm³) | 36534.9 | 31169.5 | 42133.0 |
| Parietal cerebrospinal fluid region, mean volume (mm³) | 36045.5 | 29592.5 | 42778.3 |
| Occipital cerebrospinal fluid region, mean volume (mm³) | 14828.4 | 13150.0 | 16579.6 |
| Anterior lateral ventricle region, mean volume (mm³) | 17687.3 | 13955.5 | 21581.1 |
| Posterior lateral ventricle region, mean volume (mm³) | 20476.3 | 14904.4 | 26289.8 |
| Peri-hippocampal cerebrospinal fluid region, mean volume (mm³) | 7577.0 | 5562.8 | 9678.6 |

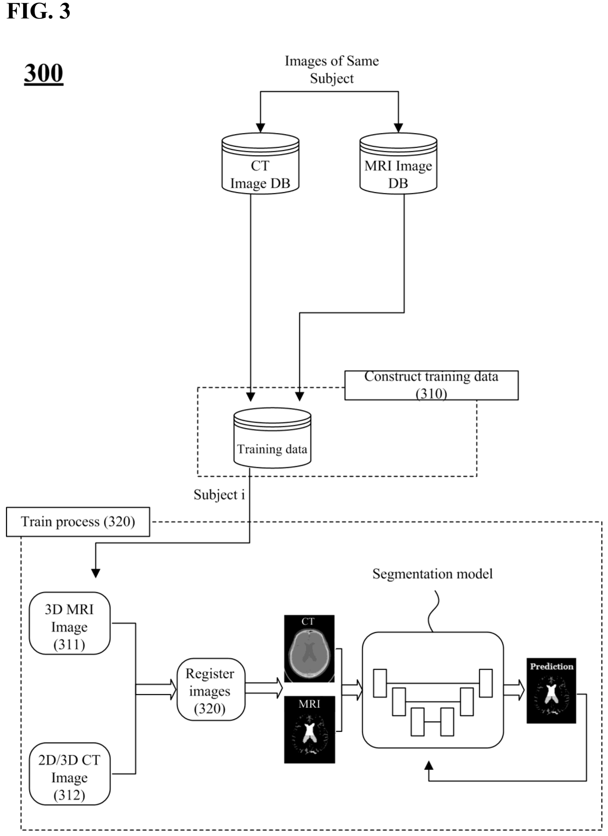

FIG. 3 is an example of a process (300) for learning a segmentation model that extracts regions of interest from brain CT images. Researchers normalized the brain CT images used as training data to a uniform size. In addition, researchers augmented the training data through brightness adjustment, horizontal flip, etc. of the training data.

Hereinafter, the training process of the model is described as being performed by a learning apparatus. Researchers constructed a segmentation model using the learning apparatus. The learning apparatus refers to a computer device capable of processing image data and performing the training process of machine learning models.

The learning apparatus learns the segmentation model using medical images of multiple subjects. However, FIG. 3 describes as an example a process of learning a segmentation model using data from subject i.

The learning apparatus constructs training data for learning the learning model (310). The training data may be constructed from brain images of a population (including dementia patients and normal people). The CT image DB (database) stores brain CT images of the population. The MRI image DB stores brain MRI images of the population. The MRI image DB includes region of interest information in brain images. The region of interest information may be a region of interest automatically or manually labeled by an expert. The learning apparatus constructs training data by extracting CT images and MRI images (including region of interest information) for the same subject.

The learning apparatus performs a process of learning a segmentation model that extracts regions of interest using the training data (320). The learning apparatus performs learning based on data for a specific patient among the training data. The learning apparatus may repeatedly perform the training process using data for multiple patients in the training data.

The learning apparatus receives subject i's 3D MRI image (including region of interest information) (311). In addition, the learning apparatus receives subject i's 2D or 3D CT image (312). The region of interest may be an ROI in a 2D image or a VOI (Volume of Interest) in a 3D image. The learning apparatus registers the brain MRI image and brain CT image (320). The learning apparatus registers images of the same brain region of the same subject.

The learning apparatus constructs a segmentation model using the registered brain CT and brain MRI. The segmentation model may be a semantic segmentation model. For example, the segmentation model may be a FCN (Fully convolutional network)-based model such as U-net.

The segmentation model predicts regions of interest based on brain CT images. In the training process, the segmentation model is trained to extract the same regions of interest in brain CT images by referring to the regions of interest in brain MRI images that are ground truth.

The regions of interest may be multiple regions as described above. The regions of interest may include frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region. The learning apparatus may construct a segmentation model for each region of interest.

Researchers also constructed multiple segmentation models for each region of interest. Researchers constructed a U-net-based segmentation model. Researchers constructed a segmentation model (2D image-based segmentation model) using each of the axial slices in the training data. In addition, researchers constructed a segmentation model (3D image-based segmentation model) using 3D CT of the training data. Table 2 below shows the results of evaluating the performance of the constructed segmentation model. Researchers performed 5-fold cross-validation on the validation data to evaluate the performance of the 2D image-based segmentation model and 3D image-based segmentation model.

**[Table 2]**

| **2D image-based segmentation model 2D (n=179)** | | | | | | | |
|---|---|---|---|---|---|---|---|
| | Frontal CSF region | Temporal CSF region | Parietal CSF region | Occipital CSF region | Anterior lateral ventricle region | Posterior lateral ventricle region | Peri-hippocampal CSF region |
| 1 Fold | 0.5686 | 0.4832 | 0.5711 | 0.4294 | 0.8848 | 0.8573 | 0.6511 |
| 2 Fold | 0.5682 | 0.4786 | 0.5686 | 0.4246 | 0.8833 | 0.8563 | 0.6476 |
| 3 Fold | 0.5815 | 0.4949 | 0.5813 | 0.4443 | 0.8816 | 0.8557 | 0.6442 |
| 4 Fold | 0.5836 | 0.4938 | 0.5835 | 0.4434 | 0.8822 | 0.8548 | 0.6435 |
| 5 Fold | 0.5711 | 0.4872 | 0.5714 | 0.4311 | 0.8849 | 0.8584 | 0.6613 |

| **3D image-based segmentation model (n=179)** | | | | | | | |
|---|---|---|---|---|---|---|---|
| | Frontal CSF region | Temporal CSF region | Parietal CSF region | Occipital CSF region | Anterior lateral ventricle region | Posterior lateral ventricle region | Peri-hippocampal CSF region |
| 1 Fold | 0.5827 | 0.4885 | 0.5903 | 0.4449 | 0.8851 | 0.8603 | 0.6278 |
| 2 Fold | 0.5865 | 0.4907 | 0.5922 | 0.448 | 0.8837 | 0.859 | 0.6273 |
| 3 Fold | 0.594 | 0.4967 | 0.6037 | 0.4608 | 0.8832 | 0.8593 | 0.6201 |
| 4 Fold | 0.6026 | 0.5023 | 0.6063 | 0.4613 | 0.8823 | 0.8563 | 0.6181 |
| 5 Fold | 0.5864 | 0.4907 | 0.594 | 0.4494 | 0.8862 | 0.8611 | 0.6357 |

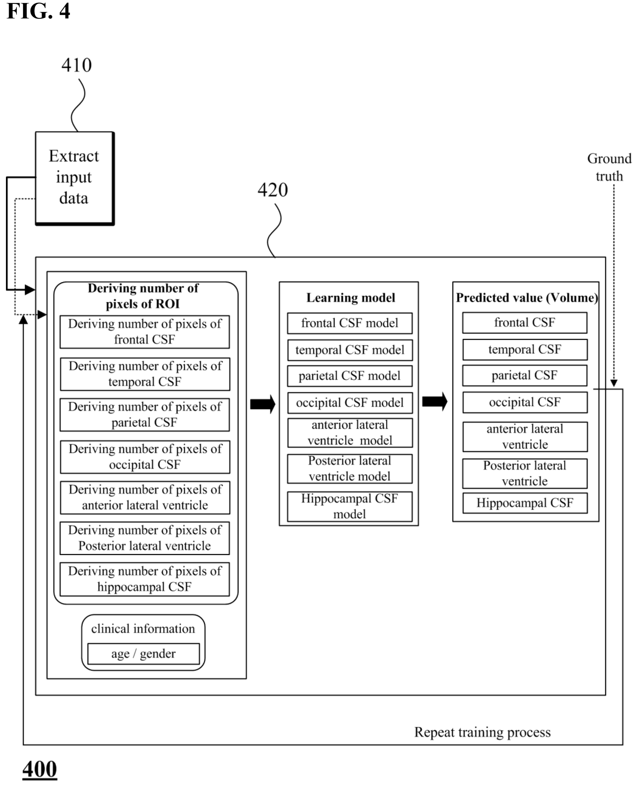

FIG. 4 is an example of a training process (400) of a learning model that predicts the volume of a region of interest based on the extracted region of interest. In FIG. 3, the learning model predicts the volume of the region of interest based on the aforementioned region of interest.

The learning in FIG. 4 presupposes a state in which the aforementioned regions of interest have been extracted from brain CT images of subjects belonging to the population. That is, the learning apparatus may use regions of interest derived using the segmentation model of FIG. 3.

The learning apparatus extracts input data based on regions of interest extracted from brain CT images (410). The input data includes region(s) of interest extracted by the segmentation model. The input data includes the actual volume (correct answer) of a specific region of interest. The actual volume may be derived from MRI. Furthermore, the input data may include clinical information (gender, age) about the subject.

The learning apparatus performs a process of learning a learning model using the extracted input data (420). The learning apparatus repeats the training process of the learning model using input data extracted from brain CT images of multiple subjects. The learning apparatus may construct a learning model that predicts volume for each region of interest.

The learning apparatus derives the total number of pixels for the region of interest. The regions of interest may be frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region, respectively, as described above.

The learning apparatus constructs a learning model by comparing the number of pixels in the region of interest with the volume (correct answer) of the corresponding region of interest. Through this process, the learning model is trained to predict the volume of the region of interest. The correct answer is the volume of a specific region of interest derived from brain MRI of subjects belonging to the population.

The learning model may be constructed as individual models according to the information to be predicted. The learning model is a machine learning model and may be implemented as one of various types of models. Researchers used a regression model. Researchers individually constructed a model for predicting the volume of frontal CSF (frontal CSF model), a model for predicting the volume of temporal CSF (temporal CSF model), a model for predicting the volume of parietal CSF (parietal CSF model), a model for predicting the volume of occipital CSF (occipital CSF model), a model for predicting the volume of anterior lateral ventricle (lateral ventricle model), a model for predicting the volume of posterior lateral ventricle (posterior lateral ventricle model), and a model for predicting the volume of peri-hippocampal cerebrospinal fluid region (hippocampal CSF model).

Furthermore, the learning model may be trained using clinical information (age and gender) in addition to the size of the corresponding region of interest.

Researchers evaluated the performance of the constructed volume prediction model.

Researchers compared the predicted volume of regions of interest extracted from brain CT images with the aforementioned segmentation model with the correct answer measured from 3D MRI. Researchers segmented regions of interest using each of the two constructed segmentation models, and compared the results of predicting volume based on these results with the ground truth. The performance evaluation results of the volume prediction model are shown in Table 3 below. The performance indicator is the Pearson Correlation Coefficient. Researchers repeated cross validation 10 times with 10 permutations. The volume of the region of interest predicted by the learning model showed significant correlation with the actual correct answer.

**[Table 3]**

| Region of Interest | **Correlation r (Volume prediction of region of interest segmented with 2D image-based)** | **Correlation r (Volume prediction of region of interest segmented with 3D image-based)** |
|---|---|---|
| Frontal cerebrospinal fluid region | 0.838 | 0.868 |
| Temporal cerebrospinal fluid region | 0.827 | 0.878 |
| Parietal cerebrospinal fluid region | 0.868 | 0.905 |
| Occipital cerebrospinal fluid region | 0.761 | 0.824 |
| Anterior lateral ventricle region | 0.984 | 0.984 |
| Posterior lateral ventricle region | 0.981 | 0.981 |
| Peri-hippocampal cerebrospinal fluid region | 0.945 | 0.944 |

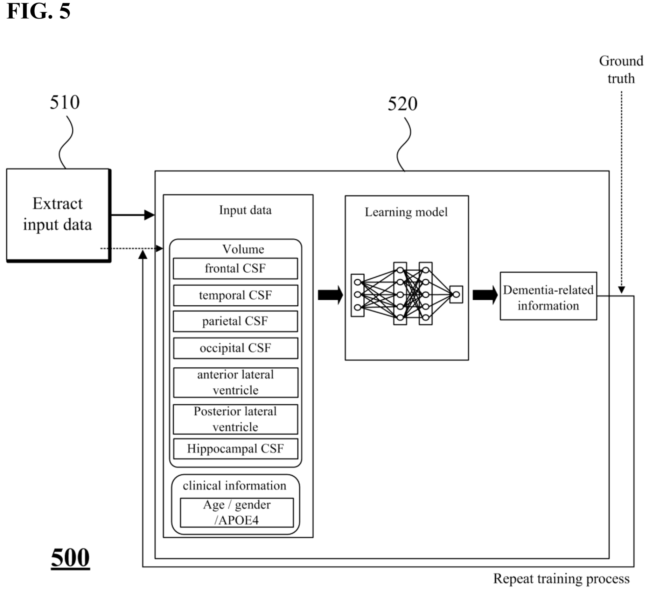

FIG. 5 is an example of a training process (500) of a learning model that derives dementia-related information based on brain region of interest volume. The learning model that derives dementia-related information can be implemented as one of various machine learning model types. Researchers implemented a model that derives dementia-related information as a deep learning model.

The learning in FIG. 5 presupposes a state in which the volume of brain regions of interest and clinical information for subjects belonging to the population have been obtained. For example, the learning apparatus may use the volume by region of interest of the subject derived using the learning model of FIG. 4.

The learning apparatus acquires input data that is training data (510).

The learning apparatus extracts the volume of a specific region of interest extracted from brain CT images of subjects belonging to the population. The brain region volume may include the volumes of frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region. Furthermore, the learning apparatus may construct a learning model to derive dementia-related information based on some regions of interest among multiple regions of interest.

In addition, the learning apparatus acquires clinical information of subjects. Clinical information may include age, gender, and APOE4 phenotype. The learning apparatus may construct a learning model to derive dementia-related information by further using some information among clinical information in addition to the volume of region(s) of interest.

The training data may include the volume of a specific brain region derived using the learning model of FIG. 3. Alternatively, the training data may include cerebral cortex thickness and volume of specific brain regions derived from actual subjects' brain images. Researchers used the volume of regions of interest derived from 3D MRI analysis results of actual subjects as training data.

The learning apparatus performs a process of learning a learning model using the extracted input data (520). The learning apparatus repeats the training process of the learning model using input data extracted from multiple subjects.

The learning apparatus performs learning by inputting the extracted input data into the learning model and comparing the value output (predicted value) by the learning model with the correct answer. The learning model is trained to predict dementia-related information of the subject. At this time, the correct answer is the dementia-related information of the corresponding subject.

Researchers constructed a learning model that classifies dementia patients or normal people. Researchers used 90% of the population data as training data and 10% of the data as validation data. The multiple learning models constructed by researchers are shown in Table 3 below. The learning models were largely constructed as models using only the volume of regions of interest (Model A), models using the volume of regions of interest and clinical information (age and gender) (Model B), and models using the volume of regions of interest and clinical information (age, gender, and APOE4 phenotype) (Model C). Each model group was constructed by dividing into models using 1, 2, 3, 4, 6, and 7 (all) regions of interest among all regions of interest.

Table 4 shows model groups using 4 different ROIs and the training data used for constructing different models in each group. In Table 4 below, the model is a model that predicts whether the subject has dementia (dementia or normal). That is, it corresponds to a model in which the learning model in FIG. 4 performs binary classification of whether the subject has dementia or is normal.

**[Table 4]**

| Model Classification | Model Name | Input Features |
|---|---|---|
| | Model A1 | 3 ROIs (aLV/pLV/HippCSF) |
| Model A | Model A2 | 4 ROIs (F/T/P/O) |
| | Model A3 | 6 ROIs (F/T/P/O/aLV/pLV) |
| | Model A4 | 7 ROIs (F/T/P/O/aLV/pLV/HippCSF) |
| | Model A5 | 2 ROIs (aLV/pLV) |
| | Model A6 | 1 ROIs (HippCSF) |
| Model B | Model B1 | 3 ROIs (aLV/pLV/HippCSF)+(age, gender) |
| | Model B2 | 4 ROIs (F/T/P/O)+(age, gender) |
| | Model B3 | 6 ROIs (F/T/P/O/aLV/pLV)+(age, gender) |
| | Model B4 | 7ROIs (F/T/P/O/aLV/pLV/HippCSF)+ (age, gender) |
| | Model B5 | 2 ROIs (aLV/pLV)+(age, gender, APOE4) |
| | Model B6 | 1 ROIs (HippCSF)+ (age, gender, APOE4) |
| Model C | Model C1 | 3 ROIs (aLV/pLV/HippCSF)+ (age, gender, APOE4) |
| | Model C2 | 4 ROIs (F/T/P/O)+ (age, gender, APOE4) |
| | Model C3 | 6 ROIs (F/T/P/O/aLV/pLV)+ (age, gender, APOE4) |
| | Model C4 | 7 ROIs (F/T/P/O/aLV/pLV/HippCSF)+ (age, gender, APOE4) |
| | Model C5 | 2 ROIs (aLV/pLV)+ (age, gender, APOE4) |
| | Model C6 | 1 ROIs (HippCSF)+ (age, gender, APOE4) |

In Table 4, regions of interest (ROI) are indicated by abbreviations. F is frontal CSF, T is temporal CSF, P is parietal CSF, O is occipital CSF, aLV is anterior lateral ventricle, pLV is posterior lateral ventricle, and HippCSF is peri-hippocampal CSF.

Researchers validated the constructed learning model. Meanwhile, in the validation process, researchers used the cerebral cortex thickness and brain region volume predicted by the learning model of FIG. 4 as input data for the learning model of FIG. 5. Researchers performed 10-fold cross-validation. The validation results are shown in Table 5 below. In Table 5, 3D CIVET is the result of classification using volume values obtained from CIVET. Table 5 shows the results of classification using regions of interest segmented with the 2D image-based segmentation model and the results of classification using regions of interest segmented with the 3D image-based segmentation model. Validation compared the results predicted by the learning model with the ground truth. Performance indicators used AUC (area under the receiver operating characteristic curve) and AUPRC (area under the precision-recall curve).

**[Table 5]**

| Model Type | 3D CIVET | | 2D image-based segmentation model classification performance | | 3D image-based segmentation model classification performance | |
|---|---|---|---|---|---|---|
| | AUC | AUPRC | AUC | AUPRC | AUC | AUPRC |
| Model A1 | 0.791 | 0.772 | 0.783 | 0.767 | 0.784 | 0.769 |
| Model A2 | 0.767 | 0.737 | 0.738 | 0.730 | 0.772 | 0.735 |
| Model A3 | 0.779 | 0.744 | 0.746 | 0.737 | 0.779 | 0.738 |
| Model A4 | 0.780 | 0.746 | 0.746 | 0.738 | 0.773 | 0.734 |
| Model A5 | 0.805 | 0.772 | 0.801 | 0.776 | 0.800 | 0.776 |
| Model A6 | 0.800 | 0.784 | 0.766 | 0.766 | 0.777 | 0.763 |
| Model B1 | 0.924 | 0.903 | 0.894 | 0.893 | 0.900 | 0.892 |
| Model B2 | 0.882 | 0.870 | 0.838 | 0.837 | 0.884 | 0.881 |
| Model B3 | 0.910 | 0.897 | 0.872 | 0.866 | 0.902 | 0.899 |
| Model B4 | 0.930 | 0.913 | 0.908 | 0.899 | 0.916 | 0.907 |
| Model B5 | 0.863 | 0.870 | 0.854 | 0.865 | 0.862 | 0.874 |
| Model B6 | 0.912 | 0.882 | 0.885 | 0.879 | 0.885 | 0.872 |
| Model C1 | 0.945 | 0.903 | 0.920 | 0.897 | 0.934 | 0.899 |
| Model C2 | 0.926 | 0.899 | 0.877 | 0.869 | 0.937 | 0.927 |
| Model C3 | 0.935 | 0.915 | 0.893 | 0.886 | 0.937 | 0.929 |
| Model C4 | 0.944 | 0.913 | 0.919 | 0.905 | 0.942 | 0.917 |
| Model C5 | 0.903 | 0.910 | 0.905 | 0.914 | 0.904 | 0.915 |
| Model C6 | 0.935 | 0.893 | 0.907 | 0.889 | 0.921 | 0.887 |

Looking at Table 5, overall, the difference in performance between the results using 3D CIVET and the results using the segmentation model was not significant. In addition, it can be seen that the results of classification using the volume of regions of interest extracted using the 2D image-based segmentation model are sufficiently significant for calculating dementia-related information. Looking at Table 5, Model A, Model B, and Model C all showed significant performance, and overall, models using additional clinical information showed slightly higher performance. Furthermore, looking at Table 5, models using anterior lateral ventricle region (aLV), posterior lateral ventricle region (pLV), and peri-hippocampal cerebrospinal fluid region (hipp CSF) had relatively higher performance.

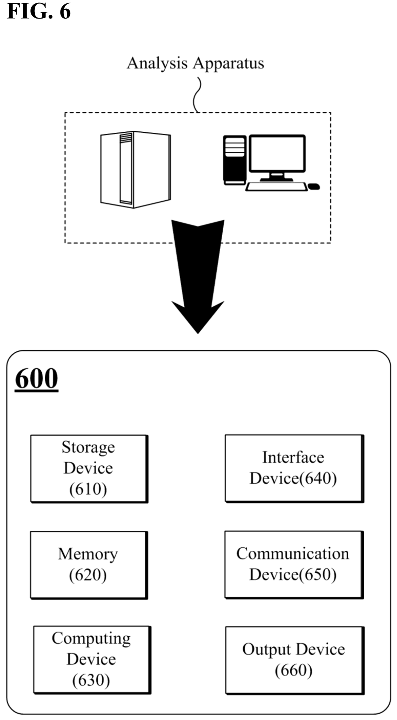

FIG. 6 is an example of an analysis apparatus (600) that derives dementia-related information of a subject using brain CT images. The analysis apparatus (600) may be physically implemented in various forms. For example, the analysis apparatus (600) may take the form of a computer device such as a PC, a smart device, a network server, or a data processing dedicated chipset. Meanwhile, the analysis apparatus (600) may be connected to brain imaging equipment or may be an integrated device.

The analysis apparatus (600) may include a storage device (610), memory (620), computing device (630), interface device (640), communication device (650), and output device (660).

The storage device (610) may store the subject's brain CT image generated by CT imaging equipment.

The storage device (610) may store the subject's clinical information. Clinical information may include at least one of age, gender, and APOE4 genotype.

The storage device (610) may store a segmentation model for extracting regions of interest from brain CT images (slices).

The storage device (610) may store a learning model that predicts the volume of the corresponding region of interest based on information about the region of interest. A learning model that predicts the volume of a specific brain region of interest is referred to as a first learning model. The region of interest includes at least one of frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region. As described above, the first learning model may be prepared for each region of interest.

In addition, the storage device (610) may store a learning model that derives dementia-related information using the volume of regions of interest. A learning model that derives dementia-related information is referred to as a second learning model. As described above, the second learning model may be implemented as various models according to the type of input data. The second learning model may be at least one of various types such as (i) a model using only the volume of regions of interest as input data (Model A), (ii) a model using the volume of regions of interest and clinical information (age, gender) as input data (Model B), or (iii) a model using the volume of regions of interest and clinical information (age, gender, and APEO4) as input data (Model C). In addition, the region of interest used by the learning model may be all 7 regions of interest or some of the regions of interest as described in Table 3.

The dementia-related information inferred by the second learning model may be at least one of information such as dementia onset status, dementia risk, dementia probability, dementia-related score, dementia prognosis prediction, degree of brain atrophy, beta-amyloid (amyloid-β, Aβ) positivity, tau protein positivity, and brain age. The dementia-related information may vary depending on the type of training data (correct answer) used by the second learning model.

The memory (620) may store data and information generated in the process of the analysis apparatus (600) calculating dementia-related information from brain CT images.

The interface device (640) is a device that receives certain commands and data from the outside. The interface device (640) may receive the subject's brain CT image from a physically connected input device or external storage device. The interface device (640) may also transmit dementia-related information predicted based on brain CT images to external objects.

The communication device (650) refers to a configuration that receives and transmits certain information through a wired or wireless network. The communication device (650) may receive the subject's brain CT image from external objects. The communication device (650) may also transmit dementia-related information predicted based on brain CT images to external objects such as user terminals.

The interface device (640) may be a device that receives data received by the communication device (650) internally.

The output device (660) is a device that outputs certain information. The output device (660) may output interfaces necessary for the data processing process, brain images, regions of interest distinguished in brain images, dementia-related information derived based on regions of interest, etc.

The computing device (630) may uniformly preprocess the subject's brain CT image. For example, the computing device (630) may normalize the brain CT image to a certain size or resolution.

The computing device (630) may extract regions of interest by inputting the subject's brain CT image into a pre-trained segmentation model. The computing device (630) may extract regions of interest by inputting the subject's brain CT image into a pre-trained segmentation model in slice units.

The region of interest may be at least one of frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region.

The computing device (630) may extract size information about the region(s) of interest. The computing device (630) may derive the total number of pixels for each region of interest. The computing device (630) may estimate the volume of the corresponding region of interest based on the number of pixels in the region of interest for each region of interest. The computing device (630) may derive the volume of the corresponding region of interest based on the number of pixels in the corresponding region of interest using the aforementioned first learning model. The computing device (630) may estimate the volume for each of multiple regions of interest using the first learning model constructed for each region of interest.

The computing device (630) may derive the subject's dementia-related information by inputting the volume of the region(s) of interest predicted by the first learning model into the second learning model. For example, the computing device (630) may derive the subject's dementia-related information by inputting the volume of at least one region among frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region into the second learning model. In addition, the computing device (630) may derive the subject's dementia-related information by further inputting clinical information (at least one of age, gender, and APOE4 genotype) in addition to the volume of the region of interest into the second learning model. This process is as described in FIG. 5.

The computing device (630) may be a device such as a processor, AP, or chip with embedded programs that processes data and performs certain operations.

In addition, the brain CT image analysis method or dementia-related information calculation method as described above can be implemented as a program (or application) including executable algorithms that can be executed on a computer. The program may be provided by being stored in a temporary or non-transitory computer readable medium.

A non-transitory readable medium refers to a medium that stores data semipermanently and can be read by a device, rather than a medium that stores data for a short moment such as registers, cache, memory, etc. Specifically, the various applications or programs described above may be stored and provided in non-transitory readable media such as CD, DVD, hard disk, Blu-ray disk, USB, memory card, ROM (read-only memory), PROM (programmable read only memory), EPROM (Erasable PROM, EPROM) or EEPROM (Electrically EPROM) or flash memory.

Temporary readable media refer to various RAMs such as Static RAM (SRAM), Dynamic RAM (DRAM), Synchronous DRAM (SDRAM), Double Data Rate SDRAM (DDR SDRAM), Enhanced SDRAM (ESDRAM), Synclink DRAM (SLDRAM), and Direct Rambus RAM (DRRAM).

This embodiment and the drawings attached to this specification merely clearly show part of the technical ideas included in the aforementioned technology, and it is obvious that all modified examples and specific embodiments that can be easily inferred by those skilled in the art within the scope of the technical ideas included in the specification and drawings of the aforementioned technology are included in the scope of rights of the aforementioned technology.

## Claims

1. A method of deriving dementia-related information using volume predicted from brain CT, comprising:
receiving, by an analysis apparatus, a brain CT (Computed Tomography) image of a subject;
extracting, by the analysis apparatus, regions of interest by inputting the brain CT image into a trained segmentation model;
predicting, by the analysis apparatus, a volume of at least one region among the regions of interest by inputting pixel information of the regions of interest into a pre-trained first learning model; and
calculating, by the analysis apparatus, dementia-related information of the subject by inputting the volume of the at least one region into a pre-trained second learning model,
wherein the regions of interest include a plurality of regions selected from frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region.

2. The method of claim 1, wherein the first learning model comprises a plurality of learning models prepared in advance for each of the regions of interest.

3. The method of claim 1, wherein the second learning model further receives at least one information among age of the subject, gender of the subject, and APOE4 genotype of the subject to derive dementia-related information of the subject.

4. The method of claim 1, wherein the dementia-related information is one of dementia onset status, dementia risk, dementia probability, dementia-related score, dementia prognosis prediction, degree of brain atrophy, beta-amyloid (amyloid-β, Aβ) positivity, tau protein positivity, and brain age.

5. The method of claim 1, wherein the segmentation model comprises a plurality of models prepared in advance for each of the regions of interest.

6. An analysis apparatus for deriving dementia-related information using volume predicted from brain CT, comprising:
an interface device for receiving a brain CT (Computed Tomography) image of a subject;
a storage device for storing a segmentation model for extracting regions of interest from a brain CT image, a first learning model for receiving brain region of interest information and predicting volume, and a second learning model for calculating dementia-related information; and
a computing device for extracting regions of interest by inputting the received brain CT image into the segmentation model, predicting a volume of at least one region among the regions of interest by inputting pixel information of the extracted regions of interest into the first learning model, and calculating dementia-related information of the subject by inputting the volume of the at least one region into the second learning model,
wherein the regions of interest include a plurality of regions selected from frontal cerebrospinal fluid region, temporal cerebrospinal fluid region, parietal cerebrospinal fluid region, occipital cerebrospinal fluid region, anterior lateral ventricle region, posterior lateral ventricle region, and peri-hippocampal cerebrospinal fluid region.

7. The analysis apparatus of claim 6, wherein the segmentation model comprises a plurality of models prepared in advance for each of the regions of interest.

8. The analysis apparatus of claim 6, wherein the first learning model comprises a plurality of learning models prepared in advance for each of the regions of interest.

9. The analysis apparatus of claim 6, wherein the second learning model further receives at least one information among age of the subject, gender of the subject, and APOE4 genotype of the subject to derive dementia-related information of the subject.

10. The analysis apparatus of claim 6, wherein the dementia-related information is one of dementia onset status, dementia risk, dementia probability, dementia-related score, dementia prognosis prediction, degree of brain atrophy, beta-amyloid (amyloid-β, Aβ) positivity, tau protein positivity, and brain age.
